# EUROPÄISCHE PATENTSCHRIFT

(11) **EP 3 107 492 B1**
(45) Veröffentlichungstag und Bekanntmachung des Hinweises auf die Patenterteilung: **19.07.2017**
(21) Anmeldenummer: 15710426.6
(22) Anmeldetag: 13.02.2015
(51) Int. Cl.: A61F 2/20, A61M 16/04, A61M 16/10

(54) **SPRECHVENTIL MIT DECKELTEIL, UMFASSEND EIN KOLBENFÖRMIGES VERSCHLUSSTEIL**
SPEECH VALVE WITH A COVER ELEMENT, COMPRISING A PISTON-SHAPED CLOSURE ELEMENT
VALVE DE PHONATION À PARTIE COUVERCLE COMPRENANT UN ÉLÉMENT DE FERMETURE SOUS FORME DE PISTON

(30) Priorität: 18.02.2014 DE 102014002064
(43) Veröffentlichungstag der Anmeldung: 28.12.2016
(73) Patentinhaber: Andreas Fahl Medizintechnik-Vertrieb GmbH, 51149 Köln (DE)
(72) Erfinder: FAHL, Andreas, 50676 Köln (DE)
(74) Vertreter: Geskes, Christoph
(86) Internationale Anmeldenummer: PCT/EP2015/000314
(87) Internationale Veröffentlichungsnummer: WO 2015/124277

(56) Entgegenhaltungen:
- EP-A1- 1 077 658
- DE-U1-202013 001 950
- DE-U1-202013 008 092

## Beschreibung

Die Erfindung betrifft ein Sprechventil für laryngektomierte oder tracheotomierte Menschen. Das Sprechventil weist ein Deckelteil, ein Gehäuseteil und ein Filterteil auf.

Sprechventile sind aus dem Stand der Technik bekannt. Diese werden z.B. an eine Tracheostomakanüle oder ein Tracheostomapflaster angebracht, damit Menschen ohne Stimmbänder mittels einer Stimmprothese sprechen können. Durch Betätigen des Sprechventils wird Luft durch eine Stimmprothese, die in einer Fistel zwischen Trachea und Ösophagus angeordnet ist, geleitet. Auch wenn die Stimmbänder noch vorhanden sind ist es notwendig, dass die Luft nicht aus der Trachealkanüle entweicht, bevor diese die Stimmbänder erreicht.

Aus der US 4,582,058A ist bekannt, ein Sprechventil mittels eines Druckimpulses, der durch die Atmung des Patienten erfolgt, zu schließen und dadurch ein Sprechen zu ermöglichen. jedoch haben sich diese Ventile als nicht besonders zuverlässig erwiesen.

Die WO 95/17138 A schlägt vor, das Sprechventil mit einer künstlichen Nase zu kombinieren. Diese künstliche Nase ist ein Filter, der Feuchtigkeit und Wärme der ausgeatmeten Luft auffängt und die in die Trachealkanüle einströmende Luft anfeuchtet und erwärmt.

Die EP 1 077 658 B1 beschreibt ein Stimmventil mit einem Filter, wobei das Stimmventil ein elastisches Gehäuse bzw. elastisches Ventilelement aufweist und das Gehäuse bzw. Ventilelement derart beispielsweise mittels Fingerdruck verformt werden kann, dass ein Gehäuseteil zur Auflage auf einem Ventilsitz kommt und das Ventil damit geschlossen wird.

DE 20 2013 001 950 U1 beschreibt eine Feucht-Wärme-Austauschvorrichtung zum Einsetzen in ein Tracheostoma, welche eine Platte in einer Atemöffnung aufweist, die zum Verschließen der Atemöffnung geeignet ist und federnd mittels eines Filters gelagert ist.

Aufgabe der vorliegenden Erfindung ist es, ein Sprechventil zur Verfügung zu stellen, welches einfach aufgebaut und kostengünstig herzustellen ist

Die Aufgabe wird erfindungsgemäß gelöst mittels eines Sprechventils nach Anspruch 1. Weitere vorteilhafte Ausgestaltungen sind der nachfolgenden Beschreibung, den Figuren sowie den Unteransprüchen zu entnehmen. Die einzelnen Merkmale der beschriebenen Ausgestaltungen sind jedoch nicht auf diese beschränkt, sondern können unter einander und mit anderen Merkmalen zu weiteren Ausgestaltungen verknüpft werden.

Es wird ein Sprechventil für Laryngektomierte oder Tracheotomierte vorgeschlagen. Das Sprechventil umfasst ein Deckelteil, ein Gehäuseteil und einen Filter. Das Deckelteil umfasst ferner ein kolbenförmiges Verschlussteil, wobei das Verschlussteil einteilig mit dem Deckelteil verbunden ist. Das Verschlußteil ist vorteilhafterweise vollständig in einem durch das Gehäuseteil gebildeten Inneren angeordnet. Das Deckelteil umfasst des Weitern ein elastisches Material, welches insbesondere gummielastische Eigenschaften hat, wobei mittels Verformens, insbesondere ein Einbeulen nach proximal zumindest eines Bereiches des Deckelteils das Sprechventil distal des Filters verschließt. Vorteilhafterweise sind Deckelteil, Filter und/oder Gehäuseteil von einander trennbar, so dass insbesondere das Deckelteil oder der Filter austauschbar ausgestaltet ist. Deckelteil, Filter und Gehäuseteil sind somit gemäß einer vorteilhaften Ausführungsform separate Bauteile. Die Rückstellkräfte, die bei einer Überführung des Sprechventils von einer zum Sprechen notwendigen Geschlossenstellung in eine Offenstellung benötigt werden, werden überwiegend, vorteilhafterweise im wesentlichen vollständig, bevorzugt vollständig, von dem aus einem elastischen Material gebildeten Deckelteil zur Verfügung gestellt. Dadurch entfallen vorteilhafterweise ansonsten notwendige Rückstellmittel wie Federn oder ähnliches. Da durch den distalen Verschluß der Filter allenfalls gering, insbsondere überwiegend nur in einem Teilbereich, der oberhalb eines durch das Gehäuseteil zur Verfügung gestellten Ventilsitz angeordnet ist, bevorzugt im wesentlichen nicht komprimiert wird, bleibt dessen Funktion des Wärme- und Feuchtigkeitsaustausches so gut wie vollständig, wenn nicht vollständig erhalten.

Die verwendeten Richtungsangaben sind gemäß der Erfindung in Bezug auf den bestimmungsgemäßen Einbau am Körper beziehungsweise an einer Trachealkanüle oder einem Tracheostomapflaster zu verstehen.

Unter dem Begriff "distal" wird im Sinne der vorliegenden Erfindung in Bezug auf ein Merkmal der erfindungsgemäßen Vorrichtung eine Anordnung bzw. Verwendung desselben fern oder auch abgewandt oder gegenüberliegend eines Tracheostomapflasters oder einer Trachealkanüle, allgemeiner einer Hautoberfläche einer Person, die insbesondere solche Befestigungsmittel für die erfindungsgemäße Vorrichtung trägt, verstanden. Ein Verschluß distal des Filters, das heißt bei Überführung des Verschlußteils aus einer Offenstellung in eine Geschlossenstellung, bedeutet, dass der Filter vollständig unterhalb des Verschlußteils, das heißt proximal, angeordnet ist. Unter dem Begriff "proximal" wird im Sinne der vorliegenden Erfindung in Bezug auf ein Merkmal der erfindungsgemäßen Vorrichtung eine Anordnung oder Verwendung desselben nah oder auch zugewandt oder benachbart eines Tracheostomapflasters oder einer Trachealkanüle, allgemeiner einer Hautoberfläche einer Person, die insbesondere solche Befestigungsmittel für die erfindungsgemäße Vorrichtung trägt, verstanden.

Der Begriff "kolbenförmig" in Bezug auf das Verschlußteil ist dahingehend zu verstehen, dass das Verschlußteil in vertikaler Richtung im Gehäuseteil sich erstreckt über eine Teilhöhe desselben, und dass durch den Kolben der Zweck erfüllt wird, eine Verschlußstellung des Sprechventils zur Erzeugung eines Sprechens zu erzielen. Durch die kolbenförmige Ausbildung wird bevorzugt der Weg zwischen einer Unterseite des Verschlußteils bzw. der Unterseite des Deckelteils und dem Ventilsitz verkürzt. Kolbenförmig im Sinne der vorliegenden Erfindung bedeutet insbesondere nicht, dass eine vertikale Erstreckung des Verschlußteils größer ist als eine horizontale desselben.

"Einteilig" im Sinne der vorliegenden Erfindung heisst nicht, dass Verschlußteil und Deckelteil materialeinheitlich ausgebildet sein müssen. Sie können beispielsweise aus unterschiedlichen Materialien hergestellt und miteinander gleich wir verbunden sein, z.B. durch Klebung, durch mechanische Mittel etc. Auch in diesem Fall bilden beide zusammen ein einziges Bauteil des erfindungsgemäßen Sprechventils, was als einteilig im Sinne der vorliegenden Erfindung anzusprechen ist.

Das vorgeschlagene Sprechventil hat den Vorteil, dass sehr wenige Bauteile zur Realisierung des Sprechventils Verwendung finden. Ein weiterer großer Vorteil des vorgeschlagenen Sprechventils ist, dass der Filter nicht oder nur unwesentlich beim Schließen des Sprechventils komprimiert wird. Die Komprimierung des Filters hat in den aus dem Stand der Technik bekannten künstlichen Nasen beziehungsweise Sprechventilen den Nachteil, dass der Filter ausgedrückt und somit seine Feuchtigkeit verliert. Weiterhin besteht grundsätzlich die Gefahr, dass das Filtermaterial in die Trachealkanüle gedrückt und im schlimmsten Fall von dem Benutzer respiriert wird.

In einer Ausgestaltung ist vorgesehen, dass das Gehäuseteil distal einen Ventilsitz umfasst, wobei das Verschlussteil bei Betätigung des Sprechventils mit dem Ventilsitz, insbesondere über dessen Unterseite, zusammenwirkt. Vorteilhafterweise wirken Sprechventil und Ventilsitz zusammen, so dass das Ventil schließt. Das eigentliche Ventil wird vom kolbenförmigen Verschlußteil bzw. Kolben und dem Ventilsitz gebildet. Der Ventilsitz ist vorzugsweise im Inneren des Gehäuseteils angeordnet.

In einer vorteilhaften Ausführungsform ist vorgesehen, dass das Gehäuseteil distal des Ventilsitzes Einströmöffnungen umfasst. Dies hat den Vorteil, dass die einströmende Luft beim Einatmen den im Gehäuseteil angeordneten Filter vollständig durchströmt, bevor diese weiter durch die zumindest eine Ausströmöffnung in z.B. eine Trachealkanüle beziehungsweise die Trachea hineinströmt. Der Ventilsitz ist vorteilhafterweise auf einer Ebene angeordnet wie das distale Ende des Filters, kann jedoch auch oberhalb oder unterhalb des distalen Endes des Filters angeordnet sein. Bei einer Anordnung unterhalb des distalen Endes des Filters erfolgt eine geringe Kompression im Wesentlichen des überstehenden Teils des Filters bei Überführung des Sprechventils aus einer Offenstellung in eine Geschlossenstellung. In einer weiteren Ausgestaltung ist vorgesehen, dass der Ventilsitz nach distal von der Oberfläche des Filters beabstandet ist. Eine weitere Variante sieht vor, dass der Ventilsitz derart im Gehäuse angeordnet ist, dass dieser proximal von dem distalen Ende des Filters beabstandet ist, so dass bei einem Verschließen des Sprechventils der Kolben den Filter teilkomprimiert. Auf die insbesondere teilweise Verdichtung des Filters während des Verschließvorgangs wird weiter unten genauer eingegangen.

In einer weiteren Ausführungsform ist vorgesehen, dass das Gehäuseteil Einströmöffnungen umfasst, die bei Betätigung des Sprechventils mittels des Verschlussteils verschließbar sind. Insbesondere ist der Kolben so im Gehäuse geführt, dass dieser bei Betätigung des Sprechventils den Luftdurchtritt der Einströmöffnungen von innerhalb des Gehäuses zumindest teilweise versperrt. Das Sprechventil wird somit bei dieser Ausführungsform durch alternative oder gemeinsam wirkende Verschlussmechanismen verschlossen: Zum einen versperrt der Kolben zumindest teilweise den Luftdurchtritt durch die Einströmöffnung. Weiterhin ist der Kolben dergestalt ausgestattet, dass dieser mit dem Ventilsitz oder dem Filter zusammenwirkt, wenn dieser in das Gehäuseteil hineingedrückt wird, so dass eine Sperrung des Luftstroms innerhalb des Gehäuses vorgenommen wird. Eine weitere Verschlusstechnik ist, dass der Kolben vorzugsweise vollflächig auf dem Filter aufliegt, insbesondere wenn kein Ventilsitz vorhanden ist, aber sein kann, bevorzugt einen Außenumfang des Filters im eingesetzten Zustand überragt, so dass ein Luftdurchtritt durch den Filter weitgehend verhindert wird. Dies hat den Vorteil, dass eine Abdichtung des Sprechventils sicher erfolgt. Im Bereich des Verschlussteils, insbesondere zentral, können alternativ oder zusätzlich eine oder mehrere Öffnungen vorgesehen sein, damit Luft durch das Ventil strömen kann. Diese werden bei einer Schließung des Ventils durch insbesondere einen Finger des Benutzers verschlossen.

Gemäß einer weiteren Ausführungsform ist vorgesehen, dass das Deckelteil auf dem Gehäuseteil lösbar angeordnet ist. Beispielsweise können für ein Gehäuseteil unterschiedliche Deckelteile vorgesehen sein, die verschiedene Eigenschaften beispielsweise im Material, in der Festigkeit und/oder in der Formgebung aufweisen. Auch können für Schmuckzwecke unterschiedliche Farben und Verzierungen vorgesehen sein, die das Deckelteil aufweist, so dass eine variable Gestaltungsmöglichkeit des Sprechventils und somit des Tracheostomas gegeben ist.

Weiterhin vorteilhaft an der Austauschbarkeit des Deckelteils ist, dass bei einer Materialermüdung des Deckelteils durch die Benutzung und somit ständige Verformung das Deckelteil ausgetauscht werden kann, wobei Gehäuse und Filter verbleiben können. Weiterhin ist vorteilhaft, dass sofern das Deckelteil lösbar am Gehäuse angeordnet ist, der Filter leicht aus dem Gehäuse entnommen und ausgetauscht werden kann. In einer Variante des Sprechventils ist vorgesehen, dass das Deckelteil mittels einer Rastverbindung auf dem Gehäuseteil angeordnet ist.

Aber auch andere Möglichkeiten einer lösbaren Verbindung, beispielsweise zur Verfügung gestellt durch eine oder mehrere Klettverbindungen oder Klebeverbindungen, sind möglich. Die Rastverbindung kann derart ausgeführt sein, dass das Deckelteil zwei, drei, vier oder mehr Rastelemente, beispielsweise in Form von Rastnasen, aufweist, die an einer Innenfläche des Deckelteiles, der Seitenwandung des Gehäuseteiles zugeordnet, angeordnet sind. Dabei kann vorgesehen sein, dass die Rastelemente einen oberen Rand des Gehäuseteiles umfassen in einem Bereich desselben, in dem Einströmöffnungen vorgesehen sind. An diesem Rand können entsprechende Aufnahmen oder Ausnehmungen oder aber auch Durchbrüche vorgesehen sein, in welche die Rastelemente des Deckelteiles einrasten. Umgekehrt können entsprechende Rastelemente auch im distalen Endbereich des Gehäuseteiles angeordnet sein, beispielsweise Rastnasen, die in entsprechende Ausnehmungen, angeordnet an der Unterseite des Deckelteiles, einrasten. Die lösbare Verbindung zwischen Deckelteil und Gehäuseteil ist vorzugsweise derart ausgestaltet, dass im Verbindungsbereich keine Luft zwischen Deckelteil und Gehäuseteil hindurch ein- oder ausströmen kann. Es kann beispielsweise vorgesehen sein, dass an der Innenseite des Deckelteils drei oder vier Rastnasen angeordnet sind, welche wiederum auf einem zur Erzielung einer hinreichend festen Verbindung auf der Innenseite des Deckelteiles angeordneten Bund oder Wulst angeordnet sind, der in seiner Dimensionierung, d.h. insbesondere in seinem Durchmesser, dem Durchmesser des Gehäuseteils entspricht, und den oberen Rand der Seitenwandung des Gehäuseteils in etwa ab- oder überdeckt. Die Rastelemente bzw. -nasen greifen bevorzugt in entsprechende Ausnehmungen am oberen Rand des Deckelteils ein, in die Ausnehmungen können umgekehrt Rastelemente bzw. -nasen am oberen Rand des Deckelteils eingreifen. Der Wulst oder Bund kann dabei nur gering beabstandet von einem zervikalen Rand des Deckelteils angeordnet sein, soweit das Deckelteil nur eine Seitenwandung des Gehäuseteils überragt. Das Deckelteil kann aber beispielsweise mit dem Gehäuseteil vernietet oder verschraubt sein, oder aber auch beispielsweise in Form einer Bajonettverschlusses mit dem Gehäuseteil verbunden sein.

Eine weitere Variante sieht vor, dass das Deckelteil auf das Gehäuseteil aufgeschraubt wird. Weiterhin sieht eine Ausgestaltung vor, dass das Deckelteil mittels eines Bajonettverschlusses auf dem Gehäuseteil aufgebracht wird. Es kann eine weitere Variante vorgesehen sein, bei der das Deckelteil mit dem Gehäuseteil verklebt oder verschweißt wird.

Die unterschiedlichen Einstellmöglichkeiten beispielsweise in der Elastizität des Materials insbesondere des gummielastischen Materials oder in der Formgebung können unterschiedliche technische Eigenschaften des Sprechventils beeinflussen. So kann beispielsweise die Rückstellkraft durch die Materialeigenschaften und die Dicke des Materials des Deckelteils als auch durch die Formgebung beeinflusst werden. In einer Ausgestaltung ist vorgesehen, dass unterschiedlich eingestellte Deckelteile zur Verfügung gestellt werden, die austauschbar an dem Gehäuseteil angeordnet werden und für verschiedene Verwendungszwecke einsetzbar sind.

Erfindungsgemäß ist das Deckelteil zumindest teilweise aus einem elastischen Material gebildet. Vorteilhafterweise ist das elastische Material des Deckelteils ein gummielastisches Material. Bevorzugt ist das elastische Material des Deckelteiles ein linear-elastisches Material. Als gummielastisches Material kann insbesondere Naturkautschuk oder aber Synthesekautschuk, insbesondere Butyl-Kautschuk, oder aber Ethylen-Propylen-Dien-Kautschuk (EPDM), eingesetzt werden. Aber auch der Einsatz von Silikonmaterialien, insbesondere medizinischen Silikonmaterialien, ist möglich. Grundsätzlich kann das gummielastische Material des Deckelteiles ein Elastomer sein, beispielsweise ein thermoelastisches Elastomer auf Olefin- oder Urethanbasis, besonders bevorzugt ein zumindest teilweise vernetztes thermoplastisches Elastomer auf Olefinbasis, ein Polyesterelastomer, ein thermoplastischer Copolyester, ein Styrolblock-Polymere oder ein thermoplastisches Copolymere. Anstatt aus einem gummielastischen Material hergestellt zu sein, welches aus einem Polymer gebildet ist, kann das Deckelteil zumindest teilweise aus einem linear-elastischem Material, insbesondere einem Metall, gebildet sein, welches eine hinreichende Elastizität aufweist. Aber auch andere Materialien, die eine hinreichende Elastizität zur Verfügung stellen, sind möglich. Besonders bevorzugt besteht das Deckelteil aus einem elastischen, bevorzugt gummielastischem Material, weist mithin kein weiteres Material auf. Das Deckelteil kann dabei eine gleichmäßige Stärke aufweisen, die Stärke kann jedoch insbesondere im Bereich des Überganges von einer horizontalen in eine vertikale Erstreckung des Deckelteiles verringert sein, und/oder ebenso in einem Bereich, durch welchen durch die Benutzer mittels eines Fingers eine Überführung des Sprechventiles aus der Offenstellung in die Geschlossenstellung und zurück erfolgt.

In einer bevorzugten Ausgestaltung ist vorgesehen, dass zumindest ein elastischer Bereich des Deckelteils als Rückstellelement fungiert. Gemäß einer Ausgestaltung ist das elastische Material des Deckelteils ein gummielastisches Material. Wird dieses eingedrückt, um den Kolben nach proximal innerhalb des Gehäuses zu verschieben und das Sprechventil somit zu schließen, bewirkt die dem gummielastische Material inhärente Rückstellkraft, dass das Sprechventil, sobald die Kraft auf das Deckelteil verringert wird, wieder öffnet. Eine weitere Ausgestaltung sieht vor, dass das elastische Material des Deckelteils ein linear elastisches Material, beispielsweise ein Metall, ist. Das Deckelteil kann aus einem oder mehreren Materialien bestehen. Insbesondere ist in einer Ausgestaltung vorgesehen, dass Materialien mit unterschiedlichen elastischen Eigenschaften vorgesehen sind und beispielsweise der Kolben ein anderes Material, insbsondere ein rigides, d.h. nicht elastisches Material, aufweist als der Rest des Deckelteils, der bevorzugt zumindest teilweise aus einem elastischen Material gebildet ist. In einer weiteren vorteilhaften Ausgestaltung ist vorgesehen, dass das Deckelteil, und somit auch das Verschlussteil, aus einem elastischen, weiter bevorzugt identischen elastischen, Material bestehen. Bevorzugt kann das Deckelteil mit dem Verschlussteil in einem 1 - Komponenten-Spritzverfahren hergestellt werden. Bei Verwendung eines identischen, elastischen Materials kann z.B. über die Wandstärke oder sonstige mechanische Mittel wie z.B. Sicken oder Versteifungsstreben, Hohlräume o.ä. die Elastizität des Deckelteils und des Verschlussteils beeinflusst werden, so dass diese trotz gleichem Material unterschiedliche Elastizitätsmodule aufweisen.

Weiterhin sieht eine Ausgestaltung vor, dass das Verschlussteil zumindest teilweise ein rigides Material umfasst, das heißt ein biegesteifes oder nicht elastisches Material, insbesondere im Bereich der Fläche, die mit dem Ventilsitz zusammenwirkt. Die Verwendung eines rigiden Materials für das Verschlussteil hat den Vorteil, dass dieses bei der Führung durch das Gehäuseteil während der Betätigung sich nicht oder nur unwesentlich verformt. Somit kann, auch wenn der Benutzer das Deckelteil nicht mittig drückt, ein vollständiger Verschluss des Sprechventils erfolgen, da das Verschlussteil nicht einseitig beziehungsweise ungleichmäßig verformt in das Gehäuseteil eindringt und einen nur unzureichenden Verschluss bewirkt. In einer weiteren Ausgestaltung ist vorgesehen, dass die Rigidität des Verschlussteils insbesondere mittels Verwendung von gummielastischen Vollmaterial erfolgt beziehungsweise durch dieses eingestellt ist. Das Verschlußteil kann einstückig mit dem Deckelteil, insbesondere auch materialeinheitlich, ausgebildet sein. Bevorzugt ist aber das kolbenförmige Verschlußteil aus einem Material mit einem geringeren Elastizitätsmodul, also einem rigideren, bevorzugt rigideren, Material, gefertigt als das Deckelteil. Besonders bevorzugt ist das kolbenförmige Verschlußteil auf seiner dem Deckelteil zugewandten Fläche mit mindestens einer Ausnehmung oder mindestens einem Vorsprung versehen. Umgekehrt ist bevorzugt auf der Unterseite des Deckelteils, welche dem Verschlußteil zugewandt ist, mindestens ein Vorsprung oder mindestens eine Ausnehmung vorgesehen. Ausnehmung und Vorsprung von Deckelteil und Verschlußteil wirken, beispielsweise durch Form- oder Kraftschluß, insbesondere durch Klebung oder mechanische Verrastung oder ähnliches, zusammen, so dass ein einstückiges Bauteil aus Deckelteil und kolbenförmigen Verschlußteil, die einteilig verbunden sind im Sinne der vorliegenden Anmeldung, erhalten ist.

Das Deckelteil ist vorteilhafterweise hut- oder kappenmäßig ausgebildet. Hierunter ist eine Ausgestaltung des Deckelteiles derart zu verstehen, dass das Deckelteil die Seitenwandung des Gehäuseteiles zumindest teilweise, bevorzugt vollständig (d.h. nach allen Seiten) überragt. Das Überragen der Seitenwandung des Gehäuseteils erfolgt dabei vorteilhafterweise in einer Richtung im Wesentlichen parallel zu einer Hautoberfläche des Körpers. Weiter bevorzugt erfolgt die Überragung der Seitenwandung des Gehäuseteils vertikal, d.h. in Richtung der vertikalen Erstreckung des Gehäuseteiles von seinem proximalen Ende zu seinem distalen Ende. Das vertikale Überragen erfolgt bevorzugt über eine Teillänge der vertikalen Erstreckung des Gehäuseteils. Das Deckelteil kann in dem die Seitenwandung des Gehäuseteils überragenden Bereich auch Öffnungen, insbesondere schlitzförmig ausgebildete, aufweisen. Öffnungen können aber auch im Verformungsbereich des Deckelteils angeordnet sein, insbesondere genau eine Öffnung, die bei Überführung in die Geschlossenstellung durch den Nutzer verschlossen wird. Diese Ausgestaltung ist insbesondere dann vergesehen, wenn das Verschlußmittel, was möglich ist, nicht mit seiner Oberseite, die der Unterseite des Deckelteils zugewandt ist, vollständig oder teilweise mit der Unterseite des Deckelteils verbunden ist, sondern insbesondere etwas beabstandet, beispielsweise durch eine Art Befestigungskreuz, in dessen Kreuzungspunkt mindestens eine Ausnehmung oder mindestens ein Vorsprung zur Befestigung an der Unterseite des Deckelteils vorgesehen ist. Das Deckelteil kann dann auf seiner Unterseite mindestens einen Vorsprung oder mindestens eine Ausnehmung aufweisen, die mit den entsprechenden Mitteln des Verschlußteils zusammenwirken. Beispielsweise kann der Vorsprung stift- oder stabförmig ausgebildet sein und die Ausnehmung als Sackloch, das bevorzugt dem Vorsprung angepasst ist. Bevorzugt erfolgt eine form- oder kraftschlüssige Verbindung zwischen Deckelteil und Verschlußteil. Das Befestigungskreuz ist dann auf einem Zylinderabschnitt angeordnet, welcher den eigentlichen Kolben bildet. Der Zylinderabschnitt ist vorteilhafterweise als kreisrunde Platte mit einer gewissen Dicke bzw. vertikalen Erstreckung augebildet.

Vorteilhafterweise weist das Deckelteil von proximal nach distal, bezogen auf die Endbereiche des Gehäuseteils, eine Höhe auf, die mindestens 30 % bis etwa 95 %, bevorzugt etwa 40 % bis etwa 95 %, weiter bevorzugt etwa 50 % bis etwa 95 %, noch weiter bevorzugt etwa 70 % bis etwa 95 %, noch weiter bevorzugt etwa 80 % bis etwa 90 % einer Höhe des Gehäuseteils entspricht. Bevorzugt weist das Deckelteil von proximal nach distal eine Höhe auf, die maximal etwa 95 %, weiter bevorzugt maximal etwa 90 %, noch weiter bevorzugt maximal etwa 80 % der Höhe des Gehäuseteils, ermittelt ausgehend von der proximalen Unterkante, die dem Körper zugewandt ist, bis zur distalen Oberkante, die vom Körper weg zeigt, der Seitenwandung derselben.

Die Höhe des Deckelteils von proximal nach distal wird bestimmt einerseits durch den zervikalen Rand, andererseits durch die distale Oberfläche desselben, die am weitesten vom Tracheostoma absteht.

Soweit im Rahmen der vorliegenden Erfindung der Begriff "etwa" verwendet wird, so ist darunter ein Toleranzbereich zu verstehen, den der angesprochene Fachmann auf dem vorliegenden Gebiet für üblich erachtet, insbesondere ein Toleranzbereich von +/- 20 %, bevorzugt +/- 10 %, weiter bevorzugt +/- 5 %, jeweils bezogen auf den in Rede stehenden Wert.

In einer bevorzugten Ausführungsform überragt das Deckelteil das Gehäuseteil sowohl lateral als auch vertikal. Weiter bevorzugt umfasst das Deckelteil einen zervikalen Rand. Der zervikale Rand, der dem Hals eines Trägers des erfindungsgemäßen Sprechventiles zugewandt ist, ist vorzugsweise abgerundet ausgebildet, so dass keine Hautirritation auftreten, wenn bei Bewegung des Benutzers ein Hautkontakt erfolgte. Insbesondere weist das Deckelteil bei lateraler Draufsicht eine runde, eine ovale oder andersartig gerundete oder eine rechteckige Formgebung auf, wobei bei einer eckigen Formgebung die Ecken vorteilhafterweise abgerundet sind. Vorteilhafterweise sind alle Kanten des Deckelteiles abgerundet, um insbesondere Verletzungen oder Hautirritierungen zu vermeiden.

Vorteil eines jeden der Merkmale, die vorstehend im Zusammenhang mit der Ausgestaltung des Deckelteiles beschrieben sind, ist eine Führung der Luft beim Einatmen am Hals entlang, so dass die Luft, bevor sie das Innere des Sprechventiles und insbesondere den Filter erreicht, vorgewärmt ist. Je nach Ausgestaltung des Deckelteiles, insbesondere im Hinblick auf die Anordnung des zervikalen Randes eher nah an der Seitenwandung des Gehäuseteiles oder eher entfernt von dieser, als auch durch die Anordnung des Deckelteiles mit dem zervikalen Rand derart, dass der zervikale Rand nahe oder etwas weiter beabstandet dem Hals des Benutzers zugeordnet ist, kann sowohl das Volumen des aufzunehmenden Luftstromes als auch die Vorwärmung desselben eingestellt werden.

Besonders vorteilhaft ist es dabei, wenn das Deckelteil distal, und dort insbesondere in einem zentralen, dem Gehäuseteil zugewandten Bereich, keine Öffnung aufweist, die einen Luftdurchtritt von distal nach proximal durch den Filter hindurch ermöglicht. Das Gehäuseteil umfasst dann vorzugsweise eine oder mehrere Einströmöffnungen in einer Seitenwandung desselben. Hierdurch wird sichergestellt, dass die Luft im Wesentlichen am Hals entlanggeführt wird, bevor sie in das Gehäuseteil eintritt. Alternativ oder zusätzlich kann jedoch auch vorgesehen sein, dass das Deckelteil mindestens eine distale Öffnung, insbesondere in einem Bereich, der dem Inneren des Gehäuseteils zugewandt ist, aufweist. Durch die mindestens eine distale Öffnung kann zumindest ein Teil der eingeatmeten Luft direkt in das Gehäuse bzw. durch den Filter dringen, so dass die Luft erfeuchtet und erwärmt in die Trachea bzw. in die Trachealkanüle eindringen kann

In einer weiteren Variante des Sprechventils ist vorgesehen, dass der Filter mit Übermaßpassung oder über Übergangspassung in dem Gehäuseteil angeordnet ist. Vorteilhafterweise hat der Filter gemäß einer Ausgestaltung kein Spiel im Gehäuse. Dies verhindert einen Bypass von ein- und ausgeatmeter Luft am Filter vorbei. Weiterhin hat der derart angepasste Filter den Vorteil, dass dieser durch den wechselnden Luftdruck nicht im Gehäuse verschoben wird. Aber auch eine Anordnung des Filters im Gehäuse unter Bildung eines Spaltes zwischen der Außenwandung und der Innenwandung des Gehäuseteiles ist möglich. Der Filter kann durch ein Gehäuseteil gehalten sein durch Einklebung oder ähnliches, aber auch beispielsweise nur durch die Quetschpassung. Es können aber auch an der Innenwandung des Gehäuseteils Haltemittel angeordnet sein, die ein Eindringen in das Filtermaterial ermöglichen. Diese können beispielsweise als kegelstumpfförmige Vorsprünge ausgestaltet sein.

Aber auch jede andere Ausgestaltung der Haltemittel ist möglich, soweit diese nur einen Halt des Filters im Gehäuseteil sicherstellen.

In einer weiteren Ausgestaltung ist vorgesehen, dass der Filter zumindest an seinem distalen Ende zumindest bereichsweise eine luftdichte Haut umfasst. Beispielsweise kann durch das Vorsehen einer luftdichten Haut gemäß einer Ausgestaltung auf das Vorsehen eines Ventilsitzes im Gehäuse verzichtet werden. Wird beispielsweise das Verschlussteil bei Betätigung des Sprechventils derart elastisch verbogen, dass dieses nicht vollständig auf dem Filter anliegt, kann die luftdichte Haut derart auf dem distalen Ende des Filters angeordnet sein, dass diese dennoch mit dem Verschlussteil zusammenwirkt und einen vollständigen Verschluss des Sprechventils bewirkt. Vorteilhafterweise ist gemäß eine Ausgestaltung vorgesehen, dass die luftdichte Haut ringförmig auf dem distalen Ende des Filters angeordnet ist. In einer weiteren vorteilhaften Ausgestaltung ist vorgesehen, dass zumindest im Bereich des distalen Endes des Filters, der keine Haut umfasst, dieser dann mittels des Verschlussteiles verschließbar ist.

Weiterhin ist in einer Ausführungsform vorgesehen, dass der Filter durch die Betätigung des Sprechventils teilkomprimiert wird. Bei einem Verschließen kann es in einer Ausgestaltung versehentlich oder absichtlich oder durch die Toleranzen des Sprechventils zu einer Teilkomprimierung des Filters kommen. Das Sprechventil ist derart ausgestaltet, dass eine Komprimierung von etwa 0 Vol.-% bis etwa 50 Vol.-%, insbesondere etwa 1 Vol.-% bis etwa 25 Vol.-%, bevorzugt etwa 1 Vol.-% bis etwa 50 Vol.-%, weiterhin bevorzugt etwa 1 Vol.-% bis etwa 10 Vol.-%, besonders bevorzugt 1 Vol.-% bis etwa 5 Vol.-% vorgesehen ist. Die Komprimierung kann beispielsweise durch das Deckelmaterial oder durch die Geometrie des Deckels eingestellt werden. Hierdurch wird dem Benutzer durch die üblicherweise aufgewendete Kraft, beispielsweise etwa 5 N bis etwa 15 N vorgegeben, wann ein Weitereindrücken nicht mehr erforderlich ist. Versucht der Benutzer ein weiteres Eindrücken des Deckelteils, kommt es gewöhnlicher Weise zu einem Unwohlsein durch den Druck auf das Tracheostoma beziehungsweise das umliegende Gewebe oder die Trachealkanüle. Das Sprechventil ist so ausgestaltet, dass dieses schon schließt, bevor die üblicherweise aufgewendete Kraft von beispielsweise etwa 5 N bis etwa 15 N erreicht ist. Weiterhin kann die Einstellung der Komprimierung des Filters durch Vorsehen des Ventilsitzes eingestellt werden. In einer weiteren Ausgestaltung ist vorgesehen, dass mittels eines am oder im Gehäuseteil angeordneten Gegenlagers eine Kompression des Filters begrenzt ist. Vorzugsweise kann das Verschlussteil nur bis zum Gegenlager in das Sprechteil hineingedrückt werden. Die Vorsehung eines Gegenlagers verhindert ein Durchdrücken des Filters in die Trachealkanüle und somit eines Respirieren des Filters. Weiterhin wird durch die nur begrenzte Kompression ein Ausdrücken der Feuchtigkeit des Filters vermindert bis verhindert. Das Gegenlager kann beispielsweise als Stab oder Wand, der oder die sich von proximal nach distal durch den Filter erstreckt und am Gehäuse in irgendeiner Form angeordnet ist, realisiert sein. Eine weitere mögliche Ausgestaltung sieht vor, dass das Gegenlager als ein zumindest teilweise umlaufender Rand ausgestaltet ist, der vorzugsweise ungleich des Ventilsitzes ist.

Weitere vorteilhafte Ausgestaltungen gehen aus den nachfolgenden Zeichnungen hervor. Die dort dargestellten Abbildungen sind jedoch nicht beschränkend auszulegen, vielmehr können die dort beschriebenen Merkmale untereinander und mit den oben beschriebenen Merkmalen zu weiteren Ausgestaltungen kombiniert werden. Des Weiteren sei darauf verwiesen, dass die der Figurenbeschreibung angegebenen Bezugszeichen den Schutzbereich der vorliegenden Erfindung nicht beschränken, sondern lediglich auf die in den Figuren bezeichneten Ausführungsbeispiele verweisen. Es zeigen:
- Fig. 1: ein Sprechventil in einer ersten Ausführungsform in einer Schnittansicht im unbetätigten Zustand; und
- Fig. 2: das Sprechventil gemäß Fig. 1 in einer Schnittansicht im betätigten Zustand;
- Fig. 3: ein Sprechventil in einer zweiten Ausführungsform in einer Schnittansicht im unbetätigten Zustand:
- Fig. 4: das Sprechventil gemäß Fig. 3 in einer Schnittansicht im betätigten Zustand; und
- Fig. 5: eine Schnittansicht entlang des Schnittes V-V gemäß Figur 4.

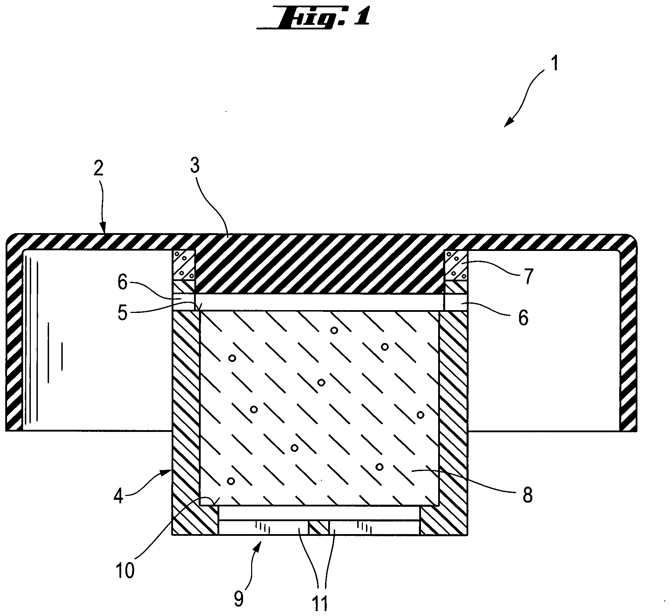

Fig. 1 zeigt ein Sprechventil 1 für laryngektomierte oder tracheotomierte Menschen mit einem Deckelteil 2, einem Gehäuseteil 4 und einem Filter 8. Das Deckelteil 2 umfasst ein kolbenförmiges Verschlussteil 3, das mit dem Deckelteil 2 verbunden ist. In der hier gezeigten Ausgestaltung ist das Deckelteil 2 vollständig aus einem Material insbesondere Silikon gefertigt. Das Deckelteil 2 ist am distalen Ende des Gehäuseteils 4 angeordnet. Vorzugsweise ist eine hier nicht weiter dargestellte Rastverbindung vorgesehen, um das Deckelteil 2 an dem Gehäuseteil 4 zu befestigen. In einer alternativen Ausgestaltung ist vorgesehen, dass das Deckelteil 2 durch festen Sitz des Verschlussteils im Gehäuseteil 4 auf dem Gehäuseteil 4 klemmt oder klebend mit diesem verbunden ist. Proximal des Verschlussteils 3 sind Einströmöffnungen 6 in der Seitenwandung des Gehäuseteils 4 vorgesehen, die ein Einströmen der Luft erlauben. Am proximalen Ende des Gehäuseteils 4 ist eine Ausströmöffnung 9 vorgesehen, die beim Einatmen ein Ausströmen der eingeatmeten Luft in die Trachealkanüle beziehungsweise das Tracheostoma ermöglicht. Ein Ventilsitz 5 ist durch die Seitenwandung des Gehäuseteils 4 als umlaufender Vorsprung in Form einer Querschnittsverengung ausgebildet.

Der Filter 8, der passgenau in dem Gehäuseteil 4 angeordnet ist, befeuchtet die einströmende Luft, bevor diese die Trachealkanüle beziehungsweise das Tracheostoma erreicht. Proximal im Gehäuseteil 4 angeordnet ist ein Filtersitz 10, der ein Rutschen des Filters in die Trachealkanüle beziehungsweise Trachea verhindert. Weiterhin sieht die hier gezeigte Ausführungsform vor, dass ein Stützkreuz 11 vorgesehen ist, welches das Eindringen von Fremdkörpern, insbesondere des Filters 8 in die Trachea ebenso verhindert.

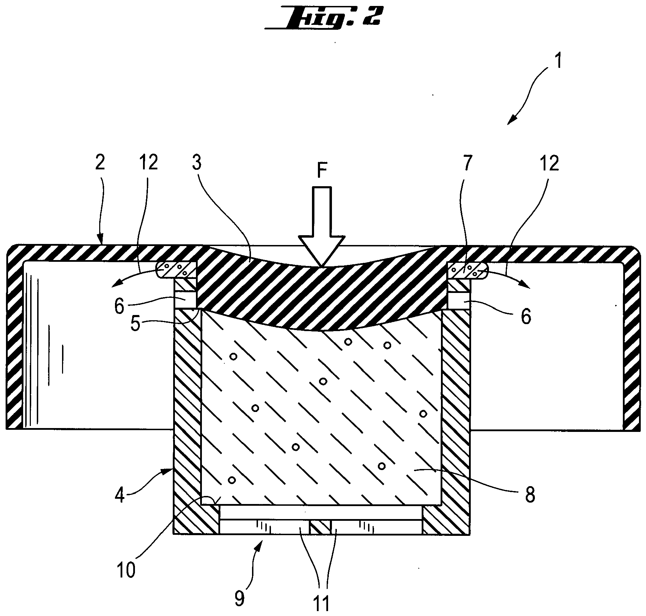

Des Weitern ist am distalen Ende des Gehäuseteils 4 angeordnet eine elastische Federung 7, welche beispielsweise aus einem offen- oder geschlossenporigen Schaumstoffmaterial oder einem sonstigen elastischen Material gebildet sein kann. Diese elastische Federung 7 ist vorzugsweise einteilig mit dem Gehäuseteil 4 verbunden und kann beispielsweise durch ZweiKomponenten-Spritzgießen mit diesem hergestellt werden. Eine derartige elastische Federung 7 kann grundsätzlich bei sämtlichen Ausführungsformen, welche in der vorliegenden Erfindung beschrieben sind, vorgesehen sein, sie muss jedoch nicht vorgesehen sein. Die Vorsehung einer elastischen Federung 7 hängt davon ab, wie das Deckelteil 2 mit dem Verschlussteil 3 ausgebildet ist, und aus welchem Material dieses gebildet ist. Gegebenenfalls kann daher auf eine elastische Federung 7 auch verzichtet werden. Fig. 2 zeigt das Sprechventil im betätigten Zustand. Eine Kraft F, die üblicherweise mittels Fingerdruck auf das Deckelteil 2 des Sprechventils 1 ausgeübt wird, verformt das Deckelteil 2 derart, dass das Verschlussteil 3 in das Gehäuseteil 4 hineinbewegt wird. Das Verschlussteil legt sich vorzugsweise vollflächig auf den Filter 8. Weiterhin ist zu erkennen, dass die Einströmöffungen 6 von dem Verschlussteil 3 innenseitig des Gehäuseteils 4 verschlossen werden. Auch ist zu erkennen, dass das Verschlussteil 3 umlaufend auf dem Ventilsitz 5 aufliegt.

In der in Fig. 2 gezeigten Geschlossenstellung des Sprechventils 1 ist ersichtlich, dass die elastische Federung 7 komprimiert wird, wobei sich Material derselben in Richtung der Pfeile 12 verschiebt und dabei über eine Außenkontur des Gehäuseteils 4 hervorragt.

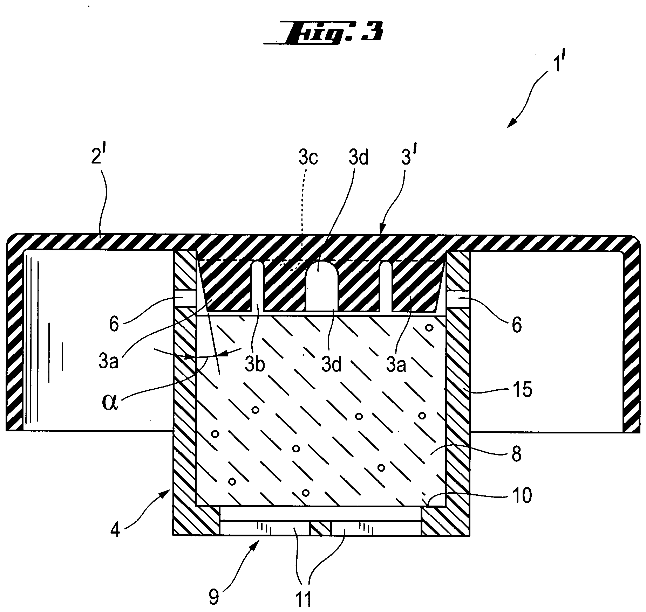

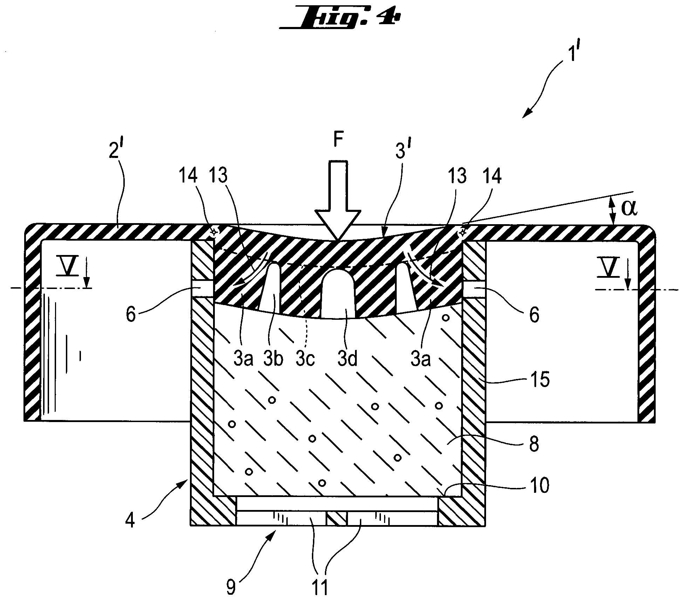

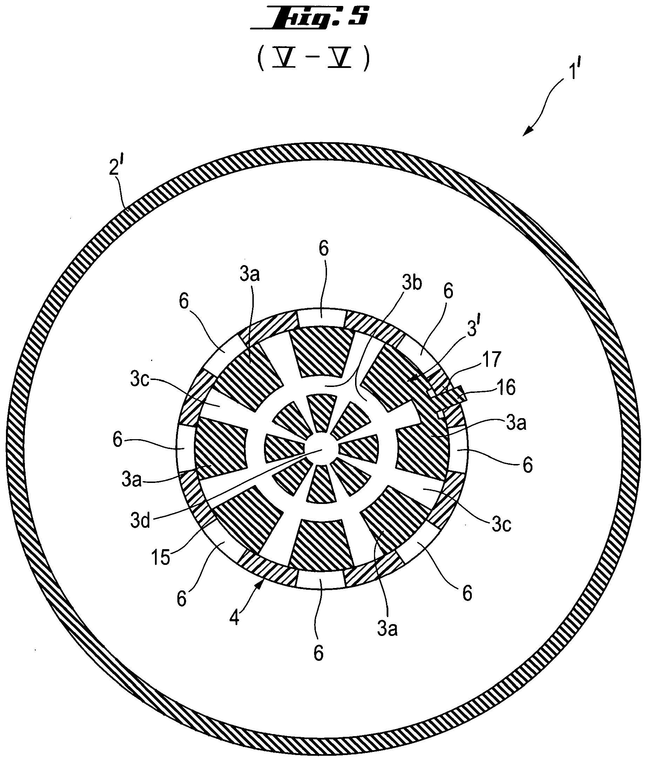

Fig. 3 zeigt nun ein Sprechventil 1' in einer weiteren, alternativen Ausgestaltung. Gleiche Merkmale werden dabei mit den gleichen Bezugszeichen bezeichnet, wie diese in den Fig. 1 und 2 verwendet wurden. Das Gehäuseteil 4 gemäß Fig. 3 ist im Wesentlichen identisch ausgebildet zu demjenigen der ersten Ausführungsform gemäß den Fig. 1 und 3, jedoch ist keine elastische Federung 7 vorgesehen. Maßgeblich unterscheidet sich die Ausführungsform der Fig. 3 in der Ausbildung eines Deckelteils 2' und eines Verschlussteiles 3', welches kolbenartig ausgebildet ist, von derjenigen des Deckelteils 2 bzw. Verschlussteils 3 gemäß der ersten Ausführungsform nach den Fig. 1 und 2. Das kolbenförmige Verschlussteil 3' weist an seinem proximalen Ende sternförmig umlaufend angeordnete Ventilverschlussklappen 3a (siehe hierzu auch Fig. 5) auf. Diese sind in Bezug auf eine Innenkontur des Gehäuseteils 4 bzw. die Innenwand des Gehäuseteils 4 mit einem sich nach proximal öffnenden Winkel α ausgebildet. Hierdurch ist es möglich, dass die der Innenwand des Gehäuseteils 4 zugewandten Seitenflächen der Ventilverschlussklappen 3a sich bei Überführung in eine Geschlossenstellung (siehe Fig. 4) an die Innenwand des Gehäuseteils 4 anlehnen und hierdurch Einströmöffnungen 6 verschließen. Diese Einströmöffnungen 6 sind vorzugsweise gleichmäßig umlaufend um den Umfang des Gehäuseteils 4 verteilt angeordnet, und dabei durch vom Gehäuseteil 4 gebildete Stege getrennt. Diesen durch das Gehäuseteil 4 gebildeten Stegen wiederum sind kreissektorartige Einschnitte 3c zugeordnet, welche dem kolbenförmigen Verschlussteil 3' eine Beweglichkeit bei Ausübung einer Kraft F bei Überführung in Geschlossenstellung vermitteln.

Des Weiteren ist im Querschnitt mindestens ein zylindrischer Einschnitt 3b vorgesehen im kolbenförmigen Verschlussteil 3', der konzentrisch um eine zentrale, sacklochförmige Ausnehmung 3d angeordnet ist, und der ebenfalls einer Anlage der Außenwand der Ventilabschlussklappen 3a an die Innenwand des Gehäuseteils 4 dient. Des Weiteren ist Fig. 3 der Winkel α zu entnehmen, der als Schwenkwinkel angesprochen werden kann und sich auf die Ventilverschlussklappen 3a bezieht, die die Einströmöffnungen 6 abdecken bei Überführung in die Geschlossenstellung nach Ausübung einer Kraft F, wie in Fig. 4 gezeigt.

Fig. 4 zeigt die zweite Ausführungsform gemäß Fig. 3 in Geschlossenstellung nach Ausübung einer Kraft F, wobei insbesondere die Verschwenkung der Ventilverschlussklappen 3a in Richtung von Pfeilen 13 und die damit erzielte Anlage der Außenwand der Ventilverschlussklappen 3a an die Innenwand des Gehäuseteils 4 verdeutlicht ist. Das kolbenförmige Verschlussteil 3' liegt dann vollflächig auf der distalen Oberseite des Filters 8 auf, der dabei minimal komprimiert wird.

Bei der Ausführungsform gemäß den Fig. 3 bis 5 ist der Ventilsitz 5 nicht mehr von Nöten bzw. kann die Anlage der Außenwand der Ventilverschlussklappen 3a an die Innenwand des Gehäuseteils 4 als Ventilsitz angesprochen werden, da hierdurch die Einströmöffnungen 6 verschlossen werden. Mithin ist die vorliegende Erfindung nicht auf solche Sprechventile beschränkt, welche einen üblichen Ventilsitz, wie in der ersten Ausführungsform gemäß den Fig. 1 und 2 gezeigt, zeigen. Im Sinne der vorliegenden Erfindung kann allgemein das Deckelteil 2 im Bereich des kolbenförmigen Verschlussteiles 3 Einschnitte jeglicher Art aufweisen, die eine Bewegbarkeit des Verschlussteiles 3 zur Verfügung stellen. Die in den Fig. 3 bis 5 gezeigte Ausführungsform ist dafürnur beispielhaft. Das Deckelteil 2 mit dem Verschlussteil 3 gemäß der ersten Ausführungsform kann beispielsweise die sacklochartige Vertiefung 3d und Einschnitte 3b aufweisen.

Fig. 5 zeigt entlang eines Schnittes V-V gemäß Fig. 4 die zweite Ausführungsform des Sprechventiles 1'. Dabei ist insbesondere gut die Anordnung der Einströmöffnungen 6 radial umlaufend, unterbrochen durch durch das Gehäuseteil 4 gebildete Stege, ersichtlich, ebenso die Zuordnung der Ventilverschlussklappen 3a zu den Einströmöffnungen 6. Ebenso ist die Ausgestaltung der zentralen sacklochförmigen Ausnehmung 3d und des konzentrisch hierzu angeordneten zylindrischen Einschnittes 3b ersichtlich. Auch sind die sektoriellen Einschnitte 3c erkennbar. Fig. 5 kann außerdem eine Arretierung des Deckelteils 2' mit dem kolbenförmigen Verschlussteil 3' entnommen werden, die dadurch verwirklicht ist, dass in einen Positionierschlitz 17 in einem zwischen zwei Einströmöffnungen 6 durch das Gehäuseteil 4 gebildeten Steg eine Positionierungsnocke 16, die am äußeren Umfang des kolbenförmigen Verschlussteiles 3' angeordnet ist, eingreift. Hierdurch wird eine Verdrehsicherung als auch Arretierung des Deckelteils 2' im Sprechventil 1' zur Verfügung gestellt. Dabei kann die Ausbildung des Positionierungsschlitzes 17 und der Positionierungsnocke 16 der Gestalt sein, dass hierdurch auch eine Rastung zur Verfügung gestellt wird, die gleichwohl eine Abnahme des einteilig ausgebildeten Deckelteils 2' vom Gehäuseteil 4 ermöglicht.

Durch die genannten Maßnahmen, aber auch einzeln durch jede für sich, wird auf einfache Weise eine sichere Abdichtung des Sprechventils 1 erreicht. Eine Rückstellkraft ist durch Auswahl des Materials, der Form und des Deckelteils bedingt und kann eingestellt werden. Wird die Kraft auf das Deckelteil gelöst beziehungsweise lässt der Benutzer von dem Deckelteil ab, führt diese Rückstellkraft zum Wiederöffnen des Ventils.

## Patentansprüche

1. Sprechventil (1) für Laryngektomierte oder Tracheotomierte mit einem Deckelteil (2), einem Gehäuseteil (4) und einem Filter (8), wobei das Deckelteil (2) ein kolbenförmiges Verschlussteil (3) umfasst und das Verschlussteil (3) einteilig mit dem Deckelteil (2) verbunden ist, wobei das Deckelteil (2) ein elastisches Material umfasst, wobei mittels Verformens zumindest eines Bereiches des Deckelteils (2) das Verschlussteil (3) das Sprechventil (1) distal des Filters (8) verschließt.

2. Sprechventil (1) nach Anspruch 1, **dadurch gekennzeichnet, dass** das Gehäuseteil (4) distal einen Ventilsitz (5) umfasst, wobei das Verschlussteil (3) bei Betätigung des Sprechventils (1) mit dem Ventilsitz (5) zusammenwirkt.

3. Sprechventil (1) nach Anspruch 2, **dadurch gekennzeichnet, dass** das Gehäuseteil (4) distal des Ventilsitzes (5) Einströmöffnungen (6) umfasst.

4. Sprechventil (1) nach einem der vorhergehenden Ansprüche, dadurch gegenzeichnet, dass das Gehäuseteil (4) Einströmöffnungen (6) distal des Filters (8) umfasst.

5. Sprechventil (1) nach einem der vorhergehenden Ansprüche, **dadurch gekennzeichnet, dass** das Gehäuseteil (4) Einströmöffnungen (6) umfasst, die bei Betätigung des Sprechventils (1) mittels des Verschlussteils (3) verschließbar sind.

6. Sprechventil (1) nach einem der vorhergehenden Ansprüche, **dadurch gekennzeichnet, dass** das Deckelteil (2) auf dem Gehäuseteil (4) lösbar angeordnet ist.

7. Sprechventil (1) nach einem der vorhergehenden Ansprüche, **dadurch gekennzeichnet, dass** das Deckelteil (2) mittels einer Rastverbindung an dem Gehäuseteil (4) angeordnet ist.

8. Sprechventil (1) nach einem der vorhergehenden Ansprüche, **dadurch gekennzeichnet, dass** zumindest ein elastischer Bereich des Deckelteils (2) als Rückstellelement fungiert.

9. Sprechventil (1) nach einem der vorhergehenden Ansprüche, **dadurch gekennzeichnet, dass** das elastische Material des Deckelteils (2) ein gummielastisches Material ist.

10. Sprechventil (1) nach einem der Ansprüche 1 bis 6, **dadurch gekennzeichnet, dass** das elastische Material des Deckelteils (2) ein linear-elastisches Material ist.

11. Sprechventil (1) nach einem der vorhergehenden Ansprüche, **dadurch gekennzeichnet, dass** das Deckelteil (2) aus dem elastischen Material besteht.

12. Sprechventil (1) nach einem der vorhergehenden Ansprüche, **dadurch gekennzeichnet, dass** das Verschlussteil (3) ein rigides Material umfasst.

13. Sprechventil (1) nach einem der vorhergehenden Ansprüche, **dadurch gekennzeichnet, dass** das Deckelteil (2) das Gehäuseteil (4) zumindest teilweise außenseitig überragt.

14. Sprechventil (1) nach einem der vorhergehenden Ansprüche, **dadurch gekennzeichnet, dass** das Deckelteil (2) das Gehäuseteil (4) lateral und vertikal überragt.

15. Sprechventil (1) nach einem der vorhergehenden Ansprüche, **dadurch gekennzeichnet, dass** das Deckelteil (2) einen zervikalen Rand (7) umfasst.

16. Sprechventil (1) nach einem der vorhergehenden Ansprüche, **dadurch gekennzeichnet, dass** das Deckelteil (2) von proximal nach distal eine Höhe umfasst, die etwa der Höhe des Gehäuseteils (4) entspricht.

17. Sprechventil (1) nach einem der vorhergehenden Ansprüche, **dadurch gekennzeichnet, dass** der Filter (8) mit Übergangspassung oder Übermaßpassung in dem Gehäuseteil (4) angeordnet ist.

18. Sprechventil (1) nach einem der vorhergehenden Ansprüche, **dadurch gekennzeichnet, dass** der Filter (8) zumindest an seinem distalen Ende zumindest bereichsweise eine luftdichte Haut umfasst.

19. Sprechventil (1) nach Anspruch 18, **dadurch gekennzeichnet, dass** zumindest ein Bereich des distalen Endes des Filters (8), der keine Haut umfasst, mittels des Verschlussteils (3) verschließbar ist.

20. Sprechventil (1) nach einem der vorhergehenden Ansprüche, **dadurch gekennzeichnet, dass** der Filter (8) durch die Betätigung des Sprechventils (1) teilkomprimiert wird.

21. Sprechventil (1) nach einem der vorhergehenden Ansprüche, **dadurch gekennzeichnet, dass** mittels eines am oder im Gehäuseteil (4) angeordneten Gegenlagers eine Kompression des Filters (8) begrenzt ist.

## Claims

1. A speech valve (1) for laryngectomy or tracheotomy patients having a cover element (2), a housing part (4) and a filter (8), wherein the cover element (2) comprises a piston-shaped closure element (3), and the closure element (3) is connected to the cover element (2) in an integral manner, wherein the cover element (2) comprises an elastic material, wherein the closure element (3) closes the speech valve (1) at the distal end to the filter (8) by deforming at least a region of the cover element (2).

2. Speech valve (1) according to claim 1, **characterized in that** the housing part (4) comprises a valve seat (5) at the distal end, wherein the closure element (3) interacts with the valve seat (5) when the speech valve (1) is actuated.

3. Speech valve (1) according to claim 2, **characterized in that** the housing part (4) comprises inflow openings (6) distal to the valve seat (5).

4. Speech valve (1) according to one of the preceding claims, **characterized in that** the housing part (4) comprises inflow openings (6) at the distal end of the filter (8).

5. Speech valve (1) according to one of the preceding claims, **characterized in that** the housing part (4) comprises inflow openings (6), which can be closed by means of the closure element (3) when the speech valve (1) is actuated.

6. Speech valve (1) according to one of the preceding claims, **characterized in that** the cover element (2) is releasably disposed on the housing part (4).

7. Speech valve (1) according to one of the preceding claims, **characterized in that** the cover element (2) is disposed on the housing part (4) by means of a latching connection.

8. Speech valve (1) according to one of the preceding claims, **characterized in that** at least one elastic region of the cover element (2) functions as a reset element.

9. Speech valve (1) according to one of the preceding claims, **characterized in that** the elastic material of the cover element (2) is a rubbery elastic material.

10. Speech valve (1) according to one of the claims 1 to 6, **characterized in that** the elastic material of the cover element (2) is a linear-elastic material.

11. Speech valve (1) according to one of the preceding claims, **characterized in that** the cover element (2) is made of the elastic material.

12. Speech valve (1) according to one of the preceding claims, **characterized in that** the closure element (3) comprises a rigid material.

13. Speech valve (1) according to one of the preceding claims, **characterized in that** the cover element (2) extends at least in part beyond the housing part (4) on the outside.

14. Speech valve (1) according to one of the preceding claims, **characterized in that** the cover element (2) extends laterally and vertically beyond the housing part (4).

15. Speech valve (1) according to one of the preceding claims, **characterized in that** the cover element (2) comprises a cervical edge (7).

16. Speech valve (1) according to one of the preceding claims, **characterized in that** the cover element (2) comprises a height from the proximal end to the distal end, which basically corresponds to the height of the housing part (4).

17. Speech valve (1) according to one of the preceding claims, **characterized in that** the filter (8) is disposed in the housing part (4) with a transition tolerance, or an excess tolerance.

18. Speech valve (1) according to one of the preceding claims, **characterized in that** the filter (8) comprises an airtight skin, at least on its distal end, at least in sections.

19. Speech valve (1) according to claim 18, **characterized in that** at least a region of the distal end of the filter (8), comprising no skin, can be closed by means of the closure element (3).

20. Speech valve (1) according to one of the preceding claims, **characterized in that** the filter (8) is partially compressed by the actuation of the speech valve (1).

21. Speech valve (1) according to one of the preceding claims, **characterized in that** a compression of the filter (8) is limited by means of a counter bearing disposed on or in the housing part (4).

## Revendications

1. Valve de phonation (1) pour laryngectomisés ou trachéotomisés avec une partie couvercle (2), une partie boîtier (4) et un filtre (8), dans laquelle la partie couvercle (2) comprend un élément de fermeture sous forme de piston (3) et l'élément de fermeture (3) est relié d'un seul tenant avec la partie couvercle (2), la partie couvercle (2) comprend un matériau élastique et l'élément de fermeture (3) obture la valve de phonation (1) de façon distale par rapport au filtre (8) par la déformation d'un moins une zone de la partie couvercle (2).

2. Valve de phonation (1) selon la revendication 1, **caractérisée en ce que** la partie boîtier (4) comprend de façon distale un siège de valve (5), l'élément de fermeture (3) coopérant avec le siège de valve (5) lors de l'activation de la valve de phonation (1).

3. Valve de phonation (1) selon la revendication 2, **caractérisée en ce que** la partie boîtier (4) comprend des orifices d'admission (6) de façon distale par rapport au siège de valve (5).

4. Valve de phonation (1) selon une des revendications précédentes, **caractérisée en ce que** la partie boîtier (4) comprend des orifices d'admission (6) de façon distale par rapport au filtre (8).

5. Valve de phonation (1) selon une des revendications précédentes, **caractérisée en ce que** la partie boîtier (4) comprend des orifices d'admission (6) qui peuvent être obturés par l'élément de fermeture (3) lors de l'activation de la valve de phonation (1).

6. Valve de phonation (1) selon une des revendications précédentes, **caractérisée en ce que** la partie couvercle (2) est montée de manière détachable sur la partie boîtier (4).

7. Valve de phonation (1) selon une des revendications précédentes, **caractérisée en ce que** la partie couvercle (2) est montée sur la partie boîtier (4) au moyen d'une fixation par encliquetage.

8. Valve de phonation (1) selon une des revendications précédentes, **caractérisée en ce qu'**au moins une zone élastique de la partie couvercle (2) fonctionne comme un élément de rappel.

9. Valve de phonation (1) selon une des revendications précédentes, **caractérisée en ce que** le matériau élastique de la partie couvercle (2) est un matériau élastique caoutchouteux.

10. Valve de phonation (1) selon une des revendications 1 à 6, **caractérisée en ce que** le matériau élastique de la partie couvercle (2) est un matériau élastique linéaire.

11. Valve de phonation (1) selon une des revendications précédentes, **caractérisée en ce que** la partie couvercle (2) est faite d'un matériau élastique.

12. Valve de phonation (1) selon une des revendications précédentes, **caractérisée en ce que** l'élément de fermeture (3) comprend un matériau rigide.

13. Valve de phonation (1) selon une des revendications précédentes, **caractérisée en ce que** la partie couvercle (2) dépasse au moins partiellement la partie boîtier (4) à l'extérieur.

14. Valve de phonation (1) selon une des revendications précédentes, **caractérisée en ce que** la partie couvercle (2) dépasse la partie boîtier (4) latéralement et verticalement.

15. Valve de phonation (1) selon une des revendications précédentes, **caractérisée en ce que** la partie couvercle (2) comprend un bord cervical (7).

16. Valve de phonation (1) selon une des revendications précédentes, **caractérisée en ce que** la partie couvercle (2) possède de la région proximale à la région distale une hauteur qui correspond approximativement à la hauteur de la partie boîtier (4).

17. Valve de phonation (1) selon une des revendications précédentes, **caractérisée en ce que** le filtre (8) est monté dans la partie boîtier (4) avec un ajustement de transition ou un ajustement par serrage.

18. Valve de phonation (1) selon une des revendications précédentes, **caractérisée en ce que** le filtre (8) comprend au moins à son extrémité distale au moins en partie une membrane étanche à l'air.

19. Valve de phonation (1) selon la revendication 18, **caractérisée en ce qu'**au moins une zone de l'extrémité distale du filtre (8) qui ne comprend pas de membrane peut être obturée au moyen de l'élément de fermeture (3).

20. Valve de phonation (1) selon une des revendications précédentes, **caractérisée en ce que** le filtre (8) est partiellement comprimé par l'activation de la valve de phonation (1).

21. Valve de phonation (1) selon une des revendications précédentes, **caractérisée en ce qu'**une compression du filtre (8) est limitée au moyen d'un contre-appui monté sur ou dans la partie boîtier (4).
